# EUROPEAN PATENT APPLICATION

(11) **EP 4 040 364 A1**
(43) Date of publication of application: **10.08.2022**
(21) Application number: 21156075.0
(22) Date of filing: 09.02.2021
(51) Int. Cl.: G06Q 30/02

(54) **AN ANALYSIS DEVICE**

(71) Applicant: Koninklijke Philips N.V., 5656 AG Eindhoven (NL)
(72) Inventor: Koop, Barbara, 5656 AE Eindhoven (NL); Everaerd, Jorus, 5656 AE Eindhoven (NL)
(74) Representative: Philips Intellectual Property & Standards

(57) **Abstract**

The invention relates to a method (600) of analyzing an experience of a product or solution. Multiple input features are defined for a type of participant. An input feature is a measurable quantity of an interaction with the product or solution, and is related to an experience driver that affects experience. Multiple training records are collected, comprising values of the multiple input features and an overall experience score. Based on the multiple training records, a machine learning model is trained to infer the experience score from at least the multiple input features. Importance weights of the input features are derived indicating an importance assigned by the machine learning model to the input feature, and output e.g. to a user as feedback about a relative importance of the respective input features on the experience.

## Description

### FIELD OF THE INVENTION

The invention relates to a method of analyzing an experience using an analysis device; to an analysis device for analyzing an experience; and to a computer readable medium.

### BACKGROUND OF THE INVENTION

Medical products and solutions are often complex systems that involve several different technical components (e.g., MRI or CT scanners, mobile measurement devices, mobile apps, central databases) with which various types of participant need to interact (e.g., medical and non-medical staff, patients, visitors, caregivers). To get the best possible outcome, medical products and solutions need to be optimized along several dimensions at the same time. This is sometimes referred to as the "quadruple aim" for value-based healthcare. The quadruple aim involves better health outcomes, lower cost of care, improved staff experience and improved patient experience.

Although listed separately, these four aims actually influence each other. For example, by improving the experience of staff with medical products, also the safety of the products can be improved, e.g., making it easier to work with a product decreases the number of errors especially in critical and stressful situations, both for professionals and for non-professionals; and it is known that health outcomes are in many cases influenced by the experience of the patient experiences, so that improving the latter can lead to an improvement in the former.

In order to be able to design products and solutions that deliver on what matters to participants interacting with them, it is important to be able to measure their experiences. In the medical sector, as in many other sectors, experiences of participants interacting with a product or solution (e.g., patient or staff experiences of a medical product or solution, or user experiences of users of any other type of product or solution) is typically measured by observing and surveying participants. For example, experiences may be measured by letting a participant take a questionnaire, and deriving a score from the questionnaire answers. For example, the system usability score (SUS) is a known such score.

### SUMMARY OF THE INVENTION

Ways of measuring that are traditionally applied to measure experiences for example in the medical sector, including observation and surveying, require either technical setup or interviews/surveys, making the exercise both intrusive and subjective. In particular, large-scale studies of experiences are typically not practically feasible due to the costs and time investments of subjects that are needed. In many cases such existing techniques also fail to get into real motivations. Another problem is that experience may be affected by many more factors, consciously and subconsciously, than can be directly influenced by product design, making it hard to interpret observation and survey results and to extract actionable data from them to deliberately steer on experience.

There is thus a need for improved techniques for analyzing and quantifying experiences, in particular techniques that provide more objective and better actionable feedback.

According to a first aspect, a method of analyzing an experience of a product or solution is provided. The method may be performed by an analysis device. To analyze an experience for a type of participant interacting with the product or solution, such as a patient or a medical professional, experience drivers that affect the experience for this type of participant, may be decomposed into measurable quantities, referred to herein as input features. Accordingly, multiple input features may be defined for the participant type, wherein an input feature may be a measurable quantity, in other words a qualitative expression, of an interaction with the product related to an experience driver.

Interestingly, the method may use a machine learning model to derive an importance of an input feature for the overall experience. To this end, multiple training records may be collected, wherein a training record may comprise values of the multiple input features and an overall experience score, e.g., an overall user experience score such as a SUS score or similar. Based on the multiple training records, a machine learning model may be trained to infer the overall experience score from at least the multiple input features. In some embodiments the machine learning model uses just the input features; in other cases the model has additional inputs. From the trained model, importance weights of the multiple input features may be derived indicating an importance assigned by the machine learning model to respective input features for inferring the overall experience score.

Determined importance weights may be output a sensory-perceptible manner to a user of the analysis system. This way, feedback may be provided to the user about a relative importance of the respective input features on the experience of the type of participant interacting with the product or solution. The user may use this feedback to monitor the performance of the product or solution and/or come up with possible improvements.

Using the provided techniques, more objective and quantitative feedback can be obtained about experiences with the product or solution. Instead of relying on surjective survey feedback of individual participants, an objective measure of the importance of particular aspects for improving the experience with the product of a type of participant generally can be obtained. Since feedback is given in terms of importance weights derived from the machine learning model, the feedback is not only more objective but also more actionable: an importance weight of an input feature indicates an improvement in experience that may be expected if that input feature is improved.

It is greatly preferred if at least some of these input features are measured automatically, e.g., are extracted from log data of the product or solution itself or measured using a measurement device external to the product or solution, without the participant under analysis needing to perform additional actions to enable the measurement, such as filling in a survey. Thus, the input feature may correspond to a target for which improvement may be comparatively easy to monitor and achieve, and for which a reasonable guarantee may be gotten that changing the value actually results in an improved experience.

This is in contrast for example to the usual practice of asking a participants' subjective opinion about particular aspects, in which case, for example, it is typically not as clear how to affect the answers and moreover, it is much less guaranteed that a change in the answer actually results in the desired change in experience for the group of participants as a whole or even the individual. For example, overall experience may affect answers to individual survey questions, but not values for particular input features. Also the fact that input features can be measured non-obtrusively in the context of regular use of the product or solution, rather than in an artificial study setting, improves objectiveness and chances that a suggested intervention has the desired effect.

Since some or all of the data can be collected automatically, it is also cheaper, and thus more feasible to collect more data and also for this reason get a more accurate and general usability assessment. In many cases, existing log data can be used so that the techniques can also be applied to products or solutions already deployed in the field. It is also possible to use survey outcomes as input features to get more complete picture. Interventions are typically directed to automatically measurable input features.

The analysis method may effectively provide a monitoring and debugging tool for the performance of the product or solution in terms of its experience, based on objective measurements. Quality of monitoring is improved. The importance weights of input features may be regarded as a debugging output uncovering features that allow experience problems to be repaired. For example, interventions to address experience may be prioritized based on the output importance weights. By measuring more directly where impact may be achieved, deliberate improvement becomes possible.

Generally, the term "participant" is used herein to refer to a person, i.e., a human being, interacting with the product or solution. Throughout this specification, participants may be users, experiences may be user experiences, and experience scores may be user experience scores. The word "participant" rather than "user" is used in this specification to stress the aspect that the participant does not need to interface directly with a user interface of the system in the computer science sense of the word. For example, in a medical imaging system, the doctor may use the user interface e.g. of an imaging workstation to interact with the system, and as such is a participant experiencing this product. However, also the patient being imaged, has an interaction in which he or she experiences the product. Both the doctor and the patient may in this example be considered to be participants, or in other words, users. The provided techniques can thus be used to analyze the experience of the patient, the doctor, or both.

Generally, participants may be divided into different types according to a role they perform, e.g., a particular person may be a participant of type doctor. The set of input features may be obtained according to a process in which first a set of experience drivers for a participant type is defined and then, per experience driver, a set of input features related to that experience driver is defined. Thus, the experience driver may be effectively decomposed to particular properties of the product or solution. Effectively, a hierarchy may thus be defined in which input features are different from drivers, and drivers are different from the overall user experience. However, in some implementations it is not needed to keep track of which experience driver an input feature is associated with, and the provided techniques can also work when drivers are not explicitly identified.

The input features are measurable, e.g., they may include objectively measurable, technical quantities such as timing measurements, values extracted from log data such as occurrence of a particular error, sensor measurements such as room temperature, control parameters such as scan speed, etcetera. Accordingly, debugging of experiences based on technical data is enabled. Thus, use patterns may be analyzed in terms of, and derived from, measurable quantities, obtaining an objective picture for a group of participants as opposed to subjective individual assessments.

Generally, input features may correspond to influenceable aspects of a product design of the product or solution. That is, it may be feasible to perform an intervention to the product or solution that is likely to change the value of the input feature. For example, an input feature may be a performance parameter, e.g., the time it takes for a certain operation to proceed, or the number of times a rescan was needed in an imaging device, for example. Input features may correspond to actions performed by the product or solution and/or participants interacting with it. Such actions are particularly suitable since they are typically automatically measurable, influenceable, and expected to have a relatively direct impact on experiences.

However, the input features used are usually not directly configurable, e.g., they are not directly adaptable as settings of the product or device, but they can rather be affected indirectly by carrying out a targeted intervention, e.g., by analyzing and optimizing performance or by modifying a design. The importance weights and other data may be output to a user of a system, allowing the user to monitoring experiences and/or initiate interventions. Such interventions may not be automatic and may involve costs and effort, but interestingly, due to the experience analysis performed, there may be increased assurance that interventions that affect experiences are worthwhile, and conversely, interventions that do not sufficiently affect experiences may be deprioritized or skipped, saving costs and effort.

However, it is also possible to apply the provided techniques in settings where an automatic intervention to an input feature is possible. For example, the output weights may be output to a system that automatically performs interventions to the input features and tests their effectiveness. For example, the system can be a system for A/B testing, also known as split-run testing, wherein the system for A/B testing uses the importance weights to prioritize interventions to input features. Since generally many variations may be possible, it is advantageous in A/B testing to select an effective intervention. The response to the intervention can be optionally measured and provided back to the analysis device as a measure of the overall experience score, e.g., for model tuning.

Each input feature may be associated with a particular experience driver. The Importance weights of input features may be reported in relation to the experience driver, e.g., the input features may be shown grouped per experience driver, or the experience driver with which an input features is associated may be otherwise output.

Optionally, the product or solution may be a medical product or solution. The participant to the product or solution whose experience is analyzed, may in such cases be a patient, or a medical practitioner (e.g., a nurse, a doctor, a surgeon etc); other participants are also possible such as a visitor to a hospital or non-medical staff at a medical institution such as a receptionist or IT expert. The medical product or solution can be a precision diagnosis product or solution, an image guided therapy product or solution, or a connected care product or solution, for example. These are types of medical settings with relatively complex interactions where experiences are particularly relevant. In particular, the product may be a medical imaging system, such as a CT scanner, an MRI scanner, or the like; a catheter; or a respiratory care device.

In some applications, the product or solution can be the set of equipment of an emergency room, for example, or a piece of personal health equipment, such as a breast pump or an oral care device. In the latter example, in particular the experience of the customer may be improved, thus leading to improved customer satisfaction, but also better health outcomes that are otherwise hard to control. However, the provided techniques are not limited to medical products or solutions.

The product can be a single device, or the solution can involve a single device, but the product or solution can also involve an assembly or installation of multiple devices, for example an assembly comprising an MRI scanner, an ultrasound measurement device, and a software portal. In such an example involving multiple devices, some or all of the participants may interact with several of the devices. Interestingly, in this case, the provided techniques allow to determine importance of interventions to respective devices, and can in particular allow to determine which of the overall devices may be prioritized for an intervention to improve experience, for example. In the medical setting, with multiple devices, it is particularly relevant to consider experiences on a care path level. To this end, at least some of the input features may be on a care path level, e.g., input features indicative of interoperability and/or operational data.

Optionally, the importance weights may be used to compute an impact score on the experience from the importance weights and values of the multiple input features. The impact score can be output to a user, for example. The impact score may indicate performance of the product or solution in terms of the input features, taking into account their relevance to experience as indicated by the importance weights. Thus, the impact score may allow to quantify and measure in an objective way a current state of user friendliness or an impact of an intervention.

In general, the values of these input features do not correspond to a training record on which the machine learning model was previously trained. The values for the input features may be actual measured values, thus allowing to monitor the performance of the product of solution in terms of experience. For example, the impact of a performed intervention can be measured. However, it is also possible to provide hypothetical values for the input features (e.g., by letting a user adjust existing values) to predict the impact of a corresponding intervention.

The inventors realized here that being able to define an exact intended result of an innovation and where success may be visible, e.g., in input features, allows to quantify and measure the impact of the innovation. In particular, by using measurements to determine an impact score, a non-intrusive and objective way is provided to support, validate and predict hypotheses on what is improved for participants in terms of experience, by providing one factor that gives an overall indication of how well we do. The impact factor can be used to steer design/innovation direction, or can help organizations to monitor and rate themselves on experience.

Although it is possible to use the overall experience score inferred by the trained machine learning model as an impact score, interestingly, the inventors realized that it is actually preferred to compute the impact score using a different formula. The impact score may be computed using the importance weights derived from the machine learning model, but without otherwise using the machine learning model.

For example, the computation may use a formula that is monotonous in the values of each of the input features, e.g., the impact factor increases as an importance weight increases or as an input feature increases. For the output inferred by a non-linear machine learning model, this is typically not the case. Here, an increase in an input feature corresponds to an improvement of the measured quantity from the experience perspective. E.g., the formula can be a linear combination of respective importance weights and input features. Such an increasing formula prevents effects where a machine learning model may for example output that if one factor is made worse, then the overall experience actually improves. It also makes it easier for a user to interactively experiment with changes to input features, since a change to an input, e.g., an increase or decrease, leads to a more predictable change in the impact score, e.g. a corresponding increase or decrease. Thus, such a formula improves usability.

A strict subset of input features may correspond to a particular experience driver that affects the experience. For example, the set of input features may be partitioned into respective non-empty sets of input features corresponding to respective experience drivers. By organizing outputs, e.g., importance weights or impact scores, along drivers, better feedback is provided to a user because the user may more readily understand experience in terms of the drivers that influence it.

Optionally, importance weights for a particular experience driver may be determined based on the importance weights of all input features together. This can be done by normalizing the importance weights for this subset of input features, e.g., such that they sum up to 1, 100, etc. These importance weights can be output to a user to enable the user to analyze that particular driver.

Optionally, given a particular driver, values may be obtained for the subset of input features relating to that driver, and an impact score for that driver may be computed from the values and importance weights for those input features. This can be done in the same way as computing the impact score as described above, e.g., as a linear combination of (normalized or non-normalized) importance weights and input features, or similar.

Optionally, the machine learning model is a non-linear model, for example an ensemble of decision trees such as gradient-boosted decision trees or a random forest. In particular, the machine learning is not a linear regression model, and the importance weights are not regression coefficients. Instead they are derived from the non-linear model. Non-linear models are more flexible than linear models. This leads to importance weights that more accurately reflect the importance of input features in respective situations. For example, a particular input feature may affect usability only in a certain subset of situations, or may even affect experience positively in some situations but negatively in other situations. A non-linear model can learn this and output an importance weight that reflects these different situations.

Another advantage of using a trainable non-linear model is that it is more generally applicable, e.g., the same type of machine learning model can be applied to many different uses with little or no manual tweaking. Non-linear models can also work well even if little data is available, and/or deal with incomplete training records that do not have values for all input features. In particular, the training may involve applying a data imputation method to the multiple training records corresponding to the type of machine learning model used. In terms of these advantages, an ensemble of decision trees was found to be particularly beneficial, but other machine learning models such as non-linear support vector machines or neural networks can also be used.

Optionally, when collecting the multiple training records, the multiple input feature may be normalized to a common scale. For example, all input features may be normalized to a 1-to-5 or 1-to-100 integer scale, to a 0-to-1 continuous scale, etcetera. In particular, for each of the input features an improvement may be represented by an increase (or decrease) in the input feature, for example. This way, the interpretability of the importance weights is improved since interventions to respective input features are comparable. Normalization may also increase the meaningfulness of a computed impact factor by ensuring that the input features it is computed from are similarly scaled. Accordingly, an aggregated quantification of impact on experience may be obtained built up out of weighed, standardized features.

The normalization is preferably defined manually based on domain knowledge. Feedback concerning the probability distributions of the input features over the multiple training records may be provided to the user (e.g., visually or as statistical parameters or an output of a statistical test) to enable the user to choose more appropriate normalizations for the input features to further improve comparability.

Optionally, the machine learning model may be tuned based on collecting additional training records. Updated importance weights may be derived from the tuned machine learning model. Interestingly, tuning enables an iterative product design process, in which interventions to the product or solution and improvements to the machine learning model may both be implemented simultaneously, allowing to intervene at an early stage yet obtain impact assessments that are as accurate as possible. In such an iterative process, for example, some or all of the training data used to train the machine learning model may initially be synthetic and may be replaced or augmented with actual training data as it is collected.

Optionally, an input feature may correspond to a number of times a given action is performed by the type of participant or by the product or solution in an interaction with the product or solution. An interaction may be a particular use of the product or solution delineated in time. For example, in medical imaging and other medical settings, an interaction may be a particular scan or a particular examination. Different features may be measured according to different interaction units, e.g., one feature may be measured per scan and another feature may be measured per scan.

Optionally, a training record may correspond to a time period. A value of an input feature and/or an experience driver and/or an overall experience score of the training record may be an aggregate value over the time period. The time period can be a day, week or month, for example. The input feature can be aggregated by averaging values for respective interactions in the time period to obtain, e.g., an average number of times an action is performed per interaction in the time period, a percentage of interactions in the time period in which a given action is performed, or another average, e.g., average temperature or average waiting time. The averaging can be done per individual or over multiple individuals in a time period, for example. Using a time period has the advantage that not all features need to be collected at the same frequency, e.g., automatic measurements may be collected per interaction and surveys may be collected for only a subset of interactions, yet they can be combined into a single record. Averaging also has the advantage of reducing the impact of outliers.

Optionally, a development over time of the values of the input features and/or the experience driver and/or the experience may be output to the user. This allows the user to track changes in experience, e.g., to investigate the effect of interventions and/or monitor whether interventions are needed.

Optionally, the machine learning model may be tested on validation records to obtain an accuracy of the machine learning model. This accuracy may be output to the user of the analysis system as an indicator of a predictability of the overall experience score from the multiple input features. Thus, the user obtains feedback on whether the right set of input features are defined for analysing experience, e.g., whether the experience drivers have been decomposed to a level of granularity which yields sufficient features to predict the overall experience score. For example, the user can add additional input features if the accuracy is insufficient. At the same time, the user may decide e.g. to remove input features with a low importance weight, e.g., that are hardly used by the machine learning model to infer the overall experience score. Thus, effectively the method of analysing experience may itself be debugged.

Optionally, the machine learning model is trained to use one or more confounding features in addition to the multiple input features for inferring the overall experience score. These are features that may be expected to affect the overall experience score while not being input features in the sense of being suitable for intervention themselves. For example, this can include environment circumstances such as whether a current medical examination is for an urgent and thus stress-inducing case (as may be derived from a patient record); what is the current room temperature (as may be measured); or what is the time of day in which an examination takes place. For example, per time unit, training records according to different values of the confounding factors may be defined.

By taking these confounding features into account, the model outputs more accurate importance weights. In some embodiments, also importance weights for the confounding features may be derived and output, but this can also be skipped to prevent clutter. In some embodiments, importance weights for input features given value(s) for one or more confounding features may be output and/or used to compute impact scores to analyse experience in different situations as indicated by the confounding features. This way, interventions to experience may be determined that lead to a more adaptive or robust product or solution.

Optionally, the collection of training records may comprise extracting values of at least one of the multiple input features from log data of the product or solution. Thus, objective data collected during the actual use is used as opposed to subjective answers.

Optionally, an input feature may represent hardware data, software data, a survey outcome, or other qualitative data. Generally, features that are automatically measurable are preferred. In particular, for such features, accurate and objective measurements may be obtained relatively efficiently. However, automatically measurable features may not provide the full picture in terms of usability and may thus need to be completed e.g. by manually collected data such as survey outcomes. Due to the use of automatic data, less non-automatic data may be needed to arrive at an accurate analysis. For example, automatically measurable data may be collected for every interaction while non-automatic data may be collected for a subset of interactions, e.g., 1 in 100 interactions or a fixed number per time period. When determining input features by aggregating per time period, such data collected with different frequencies may be combined into a single record.

A further aspect relates to an analysis device for use in analyzing an experience of a product or solution as described herein. The device may derive and output importance weights as described herein. The various advantages described for the method above, also apply to the device.

A further aspect relates to a method of using an analysis device to analyze an experience of a product or solution.

The method may comprise fixing input features for use in a machine learning model as described herein. This can be done for example, by mapping a workflow of the product or solution to identify one or more types of participant; fixing one or more experience drivers for a type of participant that affect an experience of the product or solution for the type of participant; and decomposing the drivers into input features. If needed, the method may comprise adapting the product or solution to collect log data from which input features as described herein can be extracted.

The method may comprise using an analysis device to obtain importance weights as described herein; and identifying an intervention to the product or solution based on the importance weights. The method may further comprise carrying out the intervention to improve experience. The method may further comprise computing an importance score as described herein before the intervention; computing an updated importance score after the intervention; and determining an effect of the intervention based on a difference in importance score.

A further aspect relates to a product of solution that is adapted to collect log data from which input features can be extracted for use in analyzing an experience as described herein. For example, a processor of a device of the product or solution may be adapted to collect the log data, and/or the product or solution may be provided with one or more measurement devices, e.g., sensors, to measure data from which an input feature can be extracted.

An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all the steps of an embodiment of the method when the computer program is run on a computer. Preferably, the computer program is embodied on a computer readable medium.

Another aspect of the invention provides a method of making the computer program available for downloading. This aspect is used when the computer program is uploaded into, e.g., Apple's App Store, Google's Play Store, or Microsoft's Windows Store, and when the computer program is available for downloading from such a store.

### BRIEF DESCRIPTION OF THE DRAWINGS

Further details, aspects, and embodiments of the invention will be described, by way of example only, with reference to the drawings. Elements in the figures are illustrated for simplicity and clarity and have not necessarily been drawn to scale. In the Figures, elements which correspond to elements already described may have the same reference numerals. In the drawings,
Fig. 1a schematically shows an example of an embodiment of a method of using an analysis device to analyze an experience of a product or solution;
Fig. 1b schematically shows an example of an embodiment of a model of experience;
Fig. 2 schematically shows an example an embodiment of training and using a machine learning model in analyzing an experience;
Fig. 3 schematically shows an example of an embodiment of a dashboard for displaying information about an overall experience score;
Fig. 4 schematically shows an example of an embodiment of a dashboard for displaying information about an experience driver;
Fig. 5 schematically shows an example of an embodiment of a dashboard for displaying information about multiple input features;
Fig. 6 schematically shows an example of an embodiment of a method of analyzing an experience;
Fig. 7a schematically shows a computer readable medium having a writable part comprising a computer program according to an embodiment;
Fig. 7b schematically shows an exemplary hardware diagram for implementing a device according to an embodiment.

It should be noted that items which have the same reference numbers in different Figures, have the same structural features and the same functions, or are the same signals. Where the function and/or structure of such an item has been explained, there is no necessity for repeated explanation thereof in the detailed description.

### Reference signs list

The following list of references signs is provided for facilitating the interpretation of the drawings and shall not be construed as limiting the claims.
- 101: map workflow to participants
- 102: define drivers of participant
- 103: decompose drivers into input features
- 104: define model for input features
- 105: define data sources for input features
- 106: collect data
- 107: train model from data
- 108: validate model
- 109: make model accessible to users

- 110-111: target (where impact is achieved): impact factor
- 120-122: participant, e.g., user
- 130-132: UX driver
- 140-143: touch point
- 150-153: innovation (where designing is performed)
- 160-164: data source
- 170-174: data point (where measurement is performed)

- 210: data source
- 220: training records
- 230: validation records
- 240: training machine learning model
- 250: testing machine learning model
- 260: machine learning model
- 270: deploying machine learning model
- 280: production
- 290: tuning

- 310: indicator of overall impact score
- 320: development over time of overall impact score
- 330: selection of time point
- 340: experience drivers
- 350: values of experience drivers at selected time point
- 360: importance weights of experience drivers

- 410: indicator of value of experience driver
- 420: development over time of experience driver
- 430: selection of time point
- 440: input features
- 450: values of input features at selected time point
- 460: importance weights of input features

- 511-514: values of input features
- 521-524: development over time of input features
- 530: selection of time point
- 551-554: normalized values of input features

- 600: method of analyzing experience
- 610: defining input features
- 620: collecting training records
- 630: training machine learning model
- 640: deriving importance weights
- 650: outputting importance weights

- 1000: a computer readable medium
- 1010: a writable part
- 1020: a computer program
- 1100: an analysis
- 1110: a system bus
- 1120: a processor
- 1130: a memory
- 1140: a user interface
- 1150: a communication interface
- 1160: a storage
- 1161: an operating system
- 1162-1164: instructions

### DETAILED DESCRIPTION OF THE EMBODIMENTS

While this invention is susceptible of embodiment in many different forms, there are shown in the drawings and will herein be described in detail one or more specific embodiments, with the understanding that the present disclosure is to be considered as exemplary of the principles of the invention and not intended to limit the invention to the specific embodiments shown and described. In the following, for the sake of understanding, elements of embodiments are described in operation. However, it will be apparent that the respective elements are arranged to perform the functions being described as performed by them.

Further, the invention is not limited to the embodiments, and the invention lies in each and every novel feature or combination of features described herein or recited in mutually different dependent claims.

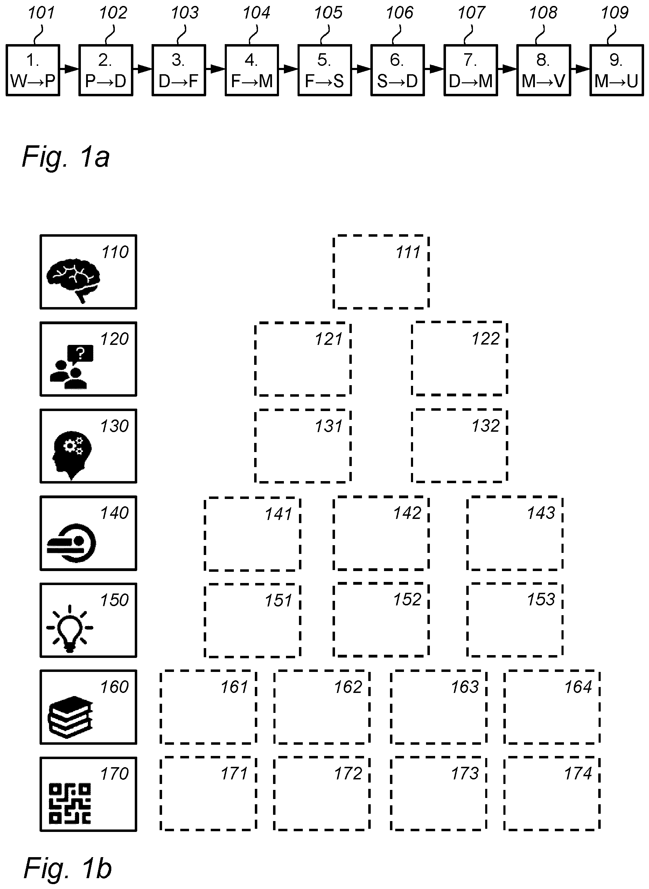

**Fig. 1a** schematically shows an example of a technique of analyzing an experience of a product or solution. The technique is referred to as the DIX ("Deliberate Impact on Experience") framework. It involves operations performed by an analysis device according to a method of analyzing an experience as described herein, and operations performed by a user of the analysis device to analyze the experience.

Generally, the term "experience" may refer to the performance of a product or solution in terms of a set of characteristics, e.g., utility, ease of use, and/or efficiency, that relate to the interaction of a type of participant with the product or solution. The experience may indicate that participant's emotions and attitudes about interacting with the product or solution. Although the experience of a single participant may be surjective, the overall experience of a representative set of participants can be regarded as an objectively measurable performance parameter of a product or solution.

Interesting, the presented techniques rely on input features, such as log data of the product or solution, to understand what happens during use of the product or solution. The input features may be related to experience drivers that affect an experience of the product or solution. The use of input features related to experience drivers may allow to measure experience more accurately. Optionally, data such as derived importance weights of input features may be organized along the corresponding experience drivers to allow a better understanding of why the measured experience is as it is.

To measure impact on experience, features that can be measured (e.g., hardware data, software data, survey outcomes and other qualitative data) may be linked to experience drivers that affect an experience, as known for example from user research about experience drivers. If it is known what drives a positive experience, e.g., for staff and patients, these experience drivers can be linked to data sources that provide measurements of corresponding input features, and from there, the experience may be measured and steered.

In an example implementation of the DIX framework, the following steps may be performed (some of which may be skipped depending on the situation):

### Operation 101: Mapping workflow W to participants P

This can be done for example, by mapping a workflow of the product or solution to identify one or more types of participant, in other words profiles of key participants. A workflow may also be referred to as a journey. A product or solution may have only one type of participant, but there can also be multiple types of participant: for example, at most 10 types of participant or at most 5 types of participant. Starting with identifying participant types may help to arrive at a profound understanding of usage patterns underlying motivations.

### Operation 102: Defining drivers D of participant P. 102

After mapping the workflow, experience drivers may be defined and fixed. An experience driver may be a factor affecting an experience of the product or solution, e.g., image quality of a medical imaging device, speed of an operation, confidence of the participant, comfort (e.g., of staff or patients), etc. Accordingly, the drivers may capture which motivations of the customer drive their experience. Drivers may be derived for each participant profile whose experience is to be analyzed. Analyzing experience in terms of drivers is advantageous since it allows to articulate a desired result, and the success with respect to that desired result, in terms of those drivers (where the impact may land or be visible). This may help to achieve deliberate impact.

Generally, in order to define experience drivers, it helps to be in the field talking to participants and performing ongoing user tests and workflow research: this way it is possible to have a good idea about what drives positive experience for them. The drivers may also be validated with the participants, e.g., in interviews. Typically, there are multiple drivers for a given participant type, for example, at least three. The number of drivers may be at most ten or, more particularly, at most five.

### Operation 103: Decomposing drivers D into input features F

Given the drivers, a set of input features may be fixed. Effectively, the drivers may be decomposed into quantifiable factors, e.g., quantification through decomposition. These input features may then be used for use in a machine learning model as described herein. For example, the input features may be determined in a workshop. A decomposition into input features may effectively represent a hypothesis on what features, or combinations features, build the individual experience drivers. These features can preferably be traced back to the properties of the product or solution, because this is where the product or solution can be affected. For example, features may be linked to actions performed by the product/solution and/or participants, e.g., patients/staff.

### Operation 104: Defining model M for input features F

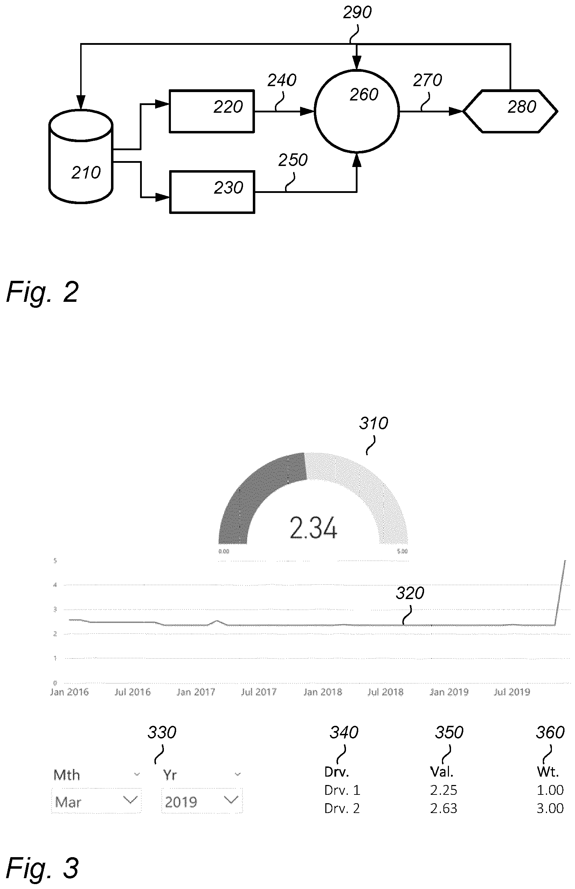

In this operation, a machine learning model may be defined for inferring an experience score from at least the multiple input features. The machine learning model can be an ensemble of decision trees, for example. Examples are discussed with respect to Fig. 2.

### Operation 105: Defining data sources S for input features F

This operation may involve investigating where data for the input features can be found, for example, by localizing the data in a database. This investigation may involve ensuring compliance to privacy regulations such as the GDPR or HIPPAA, and/or ensuring the data is of sufficient quality. If needed, the method may comprise adapting the product or solution to collect log data from which input features as described herein can be extracted.

### Operation 106: Collecting data D from sources S

The collection of training records may involve manual and/or automatic data collection. Automatic data collection may be performed by an analysis device and may comprise extracting values of input features from log data of the product or solution; measuring data using a sensor or collecting the data from an external sensor; etcetera. The data may be collected by performing monitoring over a certain time period. Data collection may involve pre-processing, cleaning, and/or standardizing the data as is known per se. In particular, input features may be normalized to a common scale that is typically user-defined. The collected data may comprise values of an overall experience score. This is typically done using surveys that may be taken using any suitable means, e.g., paper survey forms, or electronic surveys taken using a survey system that can be integrated into the product or solution or be external to it. It is also possible to use automated measurements as a proxy for overall experience, e.g., a retention rate, a success of carrying out an action, an emotion measured using an eye gaze tracker, etc.

### Operation 107: Training model M from data D

The machine learning model defined in operation 104 may be trained on the training records collected in operation 106 to infer an experience score from at least the multiple input features. Training may also be referred to as fitting the model. The fitting of the model may be performed by an analysis device as discussed herein. Based on the model, the analysis device may derive importance weights of input features for the overall experience score, as also discussed e.g. with respect to Fig. 2. The trained model can also be used to compute an impact score for input features not comprised in the training dataset, thus effectively extrapolating known combinations of input features and overall experience scores of the training dataset to this unknown input.

### Operation 108: Performing validation V of model M

The trained model may be qualitatively validated by testing the machine learning model on validation records, as also discussed with respect to Fig. 2. The model may also be validated with participants, e.g., staff, patients or other types of participant whose experience is being analysed, to make sure our outcomes and interpretations are correct.

### Operation 109: Making model M available to user U

The model may be made available to the participant, e.g., a product designer or user experience expert of the product design team, or to other stakeholders. For example, this may be in the form of a dashboard as described with respect to Fig. 3-5.

Although not shown in the figure, based on the above analysis operation, and in particular based on the importance weights that are derived and shown to a user, an intervention to a product or solution may be identified, and the intervention may be carried out to improve experience. An effect of the intervention may be determined by computing an importance score before the intervention; computing an updated importance score after the intervention; and determining an effect of the intervention based on a difference in importance score.

Some or all of the above operations may be repeated in an iterative process, e.g., operations 103-108 may be repeated until it is determined by validation 108 that an appropriate set of input features is determined, or at least operations 106 and 107 may be performed repeatedly to iteratively refine the model.

**Fig. 1b** schematically shows an example of an embodiment of a model of experience. This model shows relations between several principles underlying the technical contributions provided herein.

The figure shows the DIX (Deliberate Impact on eXperience) model being built in a bottom-up fashion. At the lowest level 170, data points are shown, e.g., four data points 171-174 in this illustration. This is where measurement is performed. One level higher, level 160, shows the data sources for the data points, illustrated by four data sources 161-164. The third level from bottom, 150, shows the innovation. This is where the designing is performed. This is illustrated by aspects 151-153 of the innovation. The fourth level 140 shows touch points for the innovation, e.g., three touch points 141-143. Still one level higher, at level 130, the experience drivers, e.g., drivers 131-132, are shown. The UX drivers relate to user or other types of participant at level 120, e.g., users 121-122. At the highest level, 110, the target 111 is illustrated. This is where the impact is achieved, as may be represented by an impact factor as described herein.

**Fig. 2** schematically shows an example an embodiment of training and using a machine learning model in analyzing an experience, as may be performed in an analysis method and/or by an analysis device.

Shown in the figure is a data source 210. From data source 210, multiple training records 220 may be collected. The number of training records can be at least 10 or at least 100. However, a relatively small number of training records may suffice, e.g., at most 500 or at most 1000 records may suffice.

A training record may comprise values of input features and an overall experience score. For example, the overall number of input features may be at least 10 and/or at most 100, e.g., at least 25 and/or at most 50. The input features may be partitioned according to which experience driver they correspond to, e.g., there may be between 2 and 25 features for an experience driver, e.g., between 5 and 10. The overall experience can be captured e.g. as a SUS (System Usability Scale) score or a NPS score (Net Promotor Score). More generally, any perceived UX or promotor score can be used, or any other score that can enable labelling of the data set.

The data source 210 can be a storage interface, a network interface, and/or a sensor interface, for example. In particular, values of at least one of the input features may be collected by extracting them from log data of the product or solution, where the log data may for example be accessed from particular the product or solution directly or from a storage where the product or solution stores the log data. Example measurements and corresponding measurement devices (e.g. part of the product or solution or of the analysis device) include:
- an eye gaze tracking device, e.g., including a camera, e.g., used to analyze how long a participant spends on an action, what their level of attention is, or their emotion;
- device logs can be used to extract e.g. durations of actions performed by participants interacting with the system, choices made by a participant, number of errors that occurs, number of tries before successfully carrying out an action, etc.;
- thermometers, pressure meters, motion detectors, etc. can be used to measure environmental conditions;
- location tracking devices such as GPS trackers or indoor location trackers can be used to e.g. to derive information (e.g., frequency, duration) about interactions among participants or between participants and the product/solution.

The training records 220 may include synthetic training records in addition to or even instead of actually measured values for the input values, at least initially. As measured data becomes available, the model may be refined based on these measurements; if sufficient real measurements are available, the synthetic records may be removed from the set of training records 220.

As an illustration, the training dataset may have the following structure:

| **date** | **\|feature 1** | **\|feature 2** | \| **...** \| | **SUS** |
|---|---|---|---|---|
| SUS collection date 1 | \| <feature 1 value> | \| <feature 2 value> | \| ... \| | <SUS val.> |
| SUS collection date 2 | \| <feature 1 value> | \| <feature 2 value> | \| ... \| | <SUS val.> |
| ... | \| ... | \| ... | | \| ... \| ... |

As also discussed elsewhere, in many cases, values of the input features and/or overall experience score may be averages, e.g., over a particular collection date or period. In particular, the feature values and/or experience score may be averaged over multiple individual participants. In a record, the number of averaged measurements may differ per input feature and/or overall experience score, e.g., allowing more measurements for input features to be used than are available for the overall experience score. Averages are indicated in the above example by "◇".

In this example, the model may be trained to infer the overall experience score, e.g., a SUS, from feature 1, feature 2, etc. Although not shown in this example, the model may use additional inputs, e.g., confounding features. Such features may not be actionable but can help to predict the overall experience more accurately and thus help to derive more accurate importance weights.

Using records 220 as a training dataset, training 240 may be performed, leading to a trained machine learning model 260. In principle, any machine learning model 260 and any training technique suitable for the type of machine learning model 260 can be used. As also discussed elsewhere it is preferred to use a non-linear model 260. For example, a neural network can be used and trained e.g. using gradient descent, e.g., stochastic gradient descent. Or, a decision tree can be used and can be trained using e.g. ID3 or C4.5. It was found that an ensemble of decision trees works particularly well as a machine learning model 260. For example, when using such an ensemble, training 240 may be done by gradient boosting. The inventors have successfully used XGBoost, but other implementations are also available and can be used.

As an optional step, missing values in training data 220 may be imputed prior to or during the training using techniques that are known per se. Gradient boosting and/or an ensemble of decision trees were found to deal with missing data particularly well.

Once trained, the model 260 may be deployed 270 into a production stage 280, where it can be used in several ways.

One way in which the model 260 can be used is by deriving importance weights of the input features from the trained model. An importance weight may indicate an importance assigned by the machine learning model to an input feature for inferring the overall experience score. This is also sometimes referred to as a feature importance. Various techniques of deriving importance weights for a trained machine learning model 260 are known per se, both general techniques and techniques that are specific to particular types of machine learning model. For example, for an ensemble of decision trees, the gain of the input feature may be used. As is known pe se, the gain may indicate the relative contribution of the corresponding feature to the model and may be calculated by taking each feature's contribution for each decision tree in the ensemble. A higher value of this metric when compared to another feature may imply that it is more important for generating a prediction.

Determining the feature importance is advantageous as it allows to get an understanding of the most contributing features. The importance weights may be output to a user. This may enable directed intervention actions to improve experience. E.g., all important weights can be output or a selection of relevant ones, e.g., the top-N or top-N% features with highest importance weights. Raw numerical importance weights as derived may be shown to the user but they can also be e.g. converted to a scale: "important"/"not important" or a more detailed scale. The weight can also be output to another system.

Another possible use of the model 260 is by using the derived importance weights to compute an impact score for a record that is not comprised in the training dataset 220: either a real record of actual measurements to determine an actually achieved impact, or a hypothetical record to predict an impact.

The impact score may be computed as a function of the importance weights and the values of the input features. In particular, at least in the case of a non-linear model the impact score may not correspond to a value as may be inferred by the machine learning model itself. As a concrete example, the impact score may be computed as a linear combination of input features and importance weights, e.g.: impact score = I_{feature1}^{∗}S_{featurel1}+I_{feature2}^{∗}S_{feature2}+I_{feature3}^{∗}S_{feature3}+I_{feature4}^{∗}S_{feature4}+I_{feature5}^{∗}S_{feature5} The impact score is also referred to as an impact factor.
An impact factor can be computed in this way for the whole system, as shown above, but also for particular drivers or features. For example, the first three features may correspond to first experience driver and the other two features may correspond to a second experience driver. Accordingly, the part I_{feature1}^{∗}S_{feature1}+I_{feature2}^{∗}S_{feature2}+I_{feature3}^{∗}S_{feature3} of the impact factor calculation relating to the first three features may be regarded as an impact factor of the first driver whereas the remaining part may be regarded as an impact factor of the second driver. Their combination may form the overall input factor. A part of the computation of the impact score corresponding to an individual feature, e.g., I_{feature1}^{∗}S_{feature1}, may be regarded as an impact factor for that feature. Impact factors may be normalized e.g. based on the number of features they are computed from.

In addition to training records 220, the data source 210 may provide validation records 230 used to assess model quality, e.g., robustness, and/or to prevent overfitting. The model may be trained on subset 220 of the data. The rest of the data 230 may be used to validate the model. It is also possible, e.g., when the number of data points is low, to use K-fold cross validation to determine the robustness of the model. In this case, training records 220 and validation records 230 at least in part coincide. An accuracy obtained by testing the machine learning model 240 on validation records 230 may be used as an indicator of the predictability of the overall experience score from the input features, allowing the user to decide to add additional features, for example.

As additional training records 220 are collected, e.g., after the model 260 has been deployed, these additional training records may be used to perform tuning 290 of the machine learning model 260. E.g., the model may be re-trained from scratch based on an updated dataset comprising the additional training records 220, or may be updated based on the additional training records. Techniques for this are known per se. As additional records 220 are collected, outdated or synthetic records that were used for previous training may be removed from the dataset. Once updated, the machine learning model 260 may be used to derive updated, and thus more accurate, importance weights that can then be output.

Figs. 3-5 show examples of how information derived from a machine learning model as described herein, may be presented to a user in the form of a dashboard. The dashboard can be generated using PowerBI or similar, for example.

The dashboard may make visible for users one or more of the following aspects:
- the overall impact score at a particular point in time,
- a development over time of the overall impact score,
- impact scores for one or more particular drivers at a particular point in time,
- a development over time of impact scores of one or more drivers,
- impact scores for one or more particular input features at a particular point in time,
- a development over time of impact scores of one or more input features,
- weights of individual input features
- a weight of a driver, e.g., determined from the weights of its corresponding input feature as a sum or similar, or extracted from the machine learning model.

The dashboard may comprise multiple screens, e.g., a screen for the overall impact score, a screen for a particular driver, and/or a screen for input features relating to a particular driver. This may allow to trace back the root cause of an impact change, all the way down to a measurement that was impact. This allows to easily check if the intended impact was made, and/or where opportunity for further improvement lies.

**Fig. 3** schematically shows an example of an embodiment of a dashboard for displaying information about an overall experience score.

The dashboard may show an indicator 310 of the overall impact factor of the product or solution, e.g. of making a MR scan. The dashboard may also show a development 320 over time of the overall impact score. The dashboard may allow to select 330 a time point for the indicator 310. The indicator may show the current overall impact score, at least by default. The dashboard may also show a list of the experience drivers 340 (e.g., image quality, enabling comfort) and/or their values 350 at the time point of the selector 330 and/or their importance weights 360. It will be understood that each of the elements 310-360 is optional and it is possible to show any subset of them. Although the dashboard shown in this figure is for a specific participant type, it is also possible to combine information for multiple participant types into a single view.

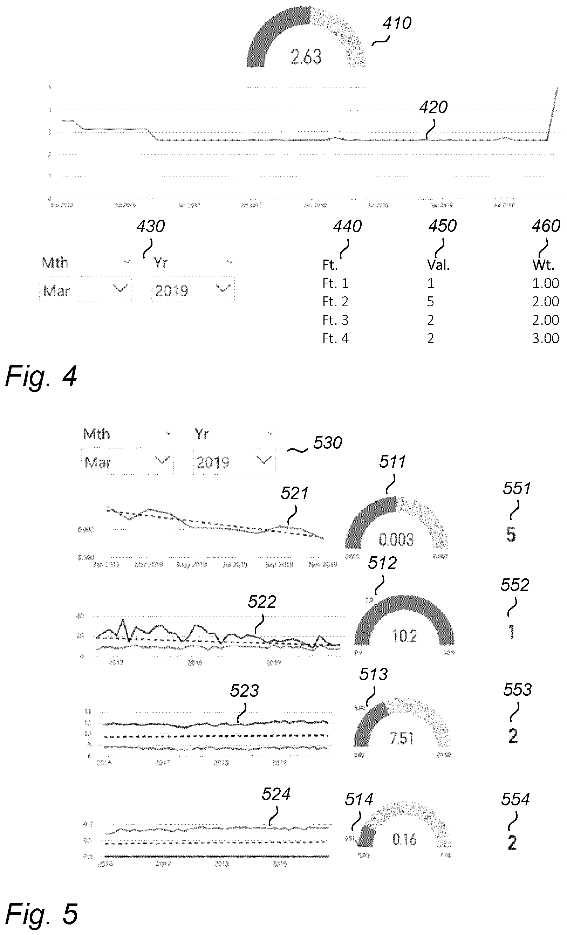

**Fig. 4** schematically shows an example of an embodiment of a dashboard for displaying information about a particular experience driver (e.g., enabling comfort). For example, the driver may be selected from the dashboard of Fig. 3.

The dashboard may show an indicator 410 of the impact score of the experience driver at a particular point in time. The dashboard may also show a development over time 420 of the experience driver. The dashboard may allow to select 430 a time point for the indicator 410. The dashboard may list input features 440 corresponding to the driver (e.g., user interaction, failed scan, table movement, and full retract table), and/or values 450 of the input features at the time point of selector 430 (in this particular example, the normalized values), and/or importance weights 460 of the input features. As above, each of these elements 410-460 is optional and any combination can be used.

**Fig. 5** schematically shows an example of an embodiment of a dashboard for displaying information about multiple input features, for example, a set of input features of a particular driver, e.g., accessed from the dashboard of Fig. 4; or the overall set of input features, e.g., accessed from the dashboard of Fig. 3. In this example, input features for a particular driver (e.g., enabling comfort) are shown.

The dashboard may show indicators 511-514 of values of respective input features (e.g., user interaction, failed scan, table movement, full retract table), e.g., four different input features are shown in this example. In this example, the indicators 511-514 shown non-normalized values and normalized values 551-554 (in this case to a scale of 1-5) are also shown. The dashboard may also allow a selection 530 of the time point for values 511-514, 551-554. The dashboard may also show a development over time 521-524 of the input features, in this case of the non-normalized values. The dashboard can also explain the features to the user, e.g., "rescan during exam means artefact which can be due to table movement", "nurse call means the patient wants to communicate", "table move out during exam means intervene in the patient comfort", etc.

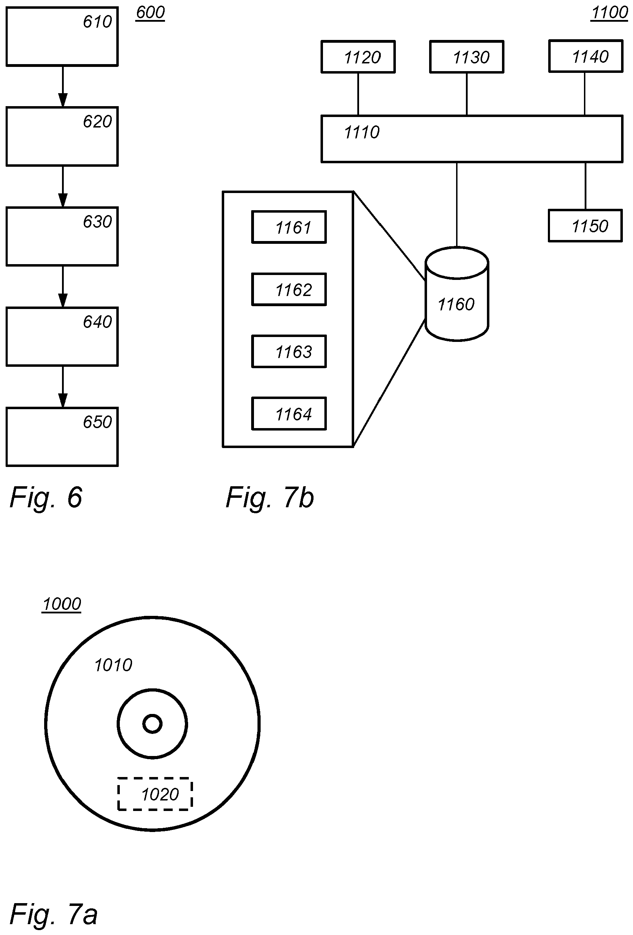

**Fig. 6** schematically shows an example of an embodiment of a method 600 of analyzing an experience of a product or solution. The method may be computer-implemented, e.g., the method 600 may be performed by an analysis device as described herein.

The method 600 may comprise, for a type of participant interacting with the product or solution, defining 610 multiple input features. An input feature may be a measurable quantity of an interaction with the product or solution. The input feature may be related to an experience driver that affects an experience of the product or solution for the type of participant.

The method 600 may comprise collecting 620 multiple training records. A training record may comprise values of the multiple input features and an overall experience score.

The method 600 may comprise, based on the multiple training records, training 630 a machine learning model to infer the experience score from at least the multiple input features.

The method 600 may comprise deriving 640 importance weights of the multiple input features from the trained machine learning model. An importance weight of an input feature may indicate an importance assigned by the machine learning model to the input feature for inferring the overall experience score.

The method 600 may comprise outputting 650 importance weights of respective input features, e.g., in a sensory-perceptible manner to a user of the analysis system, to provide feedback about a relative importance of the respective input features on the experience of the type of participant of the product or solution.

Many different ways of executing the method are possible, as will be apparent to a person skilled in the art. For example, the order of the steps can be varied or some steps may be executed in parallel. Moreover, in between steps other method steps may be inserted. The inserted steps may represent refinements of the method such as described herein, or may be unrelated to the method. Moreover, a given step may not have finished completely before a next step is started.

Embodiments of the method may be executed using software, which comprises instructions for causing a processor system to perform method 600. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the invention also extends to computer programs, particularly computer programs on or in a carrier, adapted for putting the invention into practice. The program may be in the form of source code, object code, a code intermediate source, and object code such as partially compiled form, or in any other form suitable for use in the implementation of an embodiments of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the means of at least one of the systems and/or products set forth.

**Fig. 7a** shows a computer readable medium 1000 having a writable part 1010 comprising a computer program 1020, the computer program 1020 comprising instructions for causing a processor system to perform a method of analyzing an experience, according to an embodiment. The computer program 1020 may be embodied on the computer readable medium 1000 as physical marks or by means of magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method of analyzing an experience.

**Fig. 7b** illustrates an exemplary hardware diagram 1100 for implementing an analysis device. The analysis device may be for use in analyzing an experience of a product or solution. For a type of participant interacting with the product or solution, multiple input features may be defined. An input feature may be a measurable quantity of an interaction with the product or solution. The input feature may be related to an experience driver that affects an experience of the product or solution for the type of participant.

As shown in the figure, device 1100 may comprise a memory 1130. The memory may be for storing multiple training records. A training record may comprise values of the multiple input features and an overall experience score.

The device 1100 may also comprise a processor 1120. The processor 1120 may be configured to, during operation of the device 1100, collect the multiple training records. The processor 1120 may be further configured to, based on the multiple training records, train a machine learning model to infer the experience score from at least the multiple input features. The processor 1120 may be further configured to derive importance weights of the multiple input features from the trained machine learning model. An importance weight of an input feature may indicate an importance assigned by the machine learning model to the input feature for inferring the overall experience score. The processor 1120 may be further configured to output importance weights of respective input features, e.g., in a sensory-perceptible manner to a user of the analysis system. This may be to provide feedback about a relative importance of the respective input features on the experience of the type of participant of the product or solution.

The device may also a user interface 1140, a communication interface 1150, and/or a storage 1160. The various components 1120-1160 may be interconnected via one or more system buses 1110. It will be understood that this figure constitutes, in some respects, an abstraction and that the actual organization of the components of the device 1100 may be more complex than illustrated.

The processor 1120 may be any hardware device capable of executing instructions stored in memory 1130 or storage 1160 or otherwise processing data. As such, the processor may include a microprocessor, field programmable gate array (FPGA), application-specific integrated circuit (ASIC), or other similar devices. For example, the processor may be an Intel Core i7 processor, ARM Cortex-R8, etc. In an embodiment, the processor may be ARM Cortex M0.

The memory 1130 may include various memories such as, for example LI, L2, or L3 cache or system memory. As such, the memory 1130 may include static random access memory (SRAM), dynamic RAM (DRAM), flash memory, read only memory (ROM), or other similar memory devices. It will be apparent that, in embodiments where the processor includes one or more ASICs (or other processing devices) that implement one or more of the functions described herein in hardware, the software described as corresponding to such functionality in other embodiments may be omitted.

The user interface 1140 may include one or more devices for enabling communication with a user such as an administrator. For example, the user interface 1140 may include a display, a mouse, and a keyboard for receiving user commands. In some embodiments, the user interface 1140 may include a command line interface or graphical user interface that may be presented to a remote terminal via the communication interface 1150. User interface 1140 may be used for outputting the importance weights and/or other information based on it, e.g., via a dashboard.

The communication interface 1150 may include one or more devices for enabling communication with other hardware devices. For example, the communication interface 1150 may include a network interface card (NIC) configured to communicate according to the Ethernet protocol. For example, the communication interface 1150 may comprise an antenna, connectors or both, and the like. Additionally, the communication interface 1150 may implement a TCP/IP stack for communication according to the TCP/IP protocols. Various alternative or additional hardware or configurations for the communication interface 1150 will be apparent. For example, the interface 1150 may be a network interface to a local or wide area network, e.g., the Internet, a storage interface to an internal or external data storage, a keyboard, an application interface (API), etc. The communication interface 1150 may be used for example for accessing log data of the product or solution.

The storage 1160 may include one or more machine-readable storage media such as read-only memory (ROM), random-access memory (RAM), magnetic disk storage media, optical storage media, flash-memory devices, or similar storage media. In various embodiments, the storage 1160 may store instructions for execution by the processor 1120 or data upon with the processor 1120 may operate. For example, the storage 1160 may store a base operating system 1161 for controlling various basic operations of the hardware 1100. The storage may also store instructions 1162-1164 for collecting training records, training a machine learning model, deriving importance weights, outputting importance weights, etc.

It will be apparent that various information described as stored in the storage 1160 may be additionally or alternatively stored in the memory 1130. In this respect, the memory 1130 may also be considered to constitute a "storage device" and the storage 1160 may be considered a "memory." Various other arrangements will be apparent. Further, the memory 1130 and storage 1160 may both be considered to be "non-transitory machine-readable media." As used herein, the term "non-transitory" will be understood to exclude transitory signals but to include all forms of storage, including both volatile and non-volatile memories.

While device 1100 is shown as including one of each described component, the various components may be duplicated in various embodiments. For example, the processor 1120 may include multiple microprocessors that are configured to independently execute the methods described herein or are configured to perform steps or subroutines of the methods described herein such that the multiple processors cooperate to achieve the functionality described herein. Further, where the device 1100 is implemented in a cloud computing system, the various hardware components may belong to separate physical systems. For example, the processor 1120 may include a first processor in a first server and a second processor in a second server.

The execution of the analysis device may be implemented in a processor circuit, examples of which are shown herein. In an embodiment, the analysis device comprises a training record collection circuit implementing the collection of training records; a machine learning model training circuit implementing the training of a machine learning model, an importance weight derivation circuit implementing the derivation of importance weights, and/or an outputting circuit implementing the outputting of importance weights. The device 100 may comprise additional circuits, e.g., an impact score computation circuit implementing the computation of an impact score; a normalizing circuit implementing normalization of input features to a common scale, etc.

The circuits may be a processor circuit and storage circuit, the processor circuit executing instructions represented electronically in the storage circuits.

It should be noted that the above-mentioned embodiments illustrate rather than limit the invention, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A method (600) of analyzing an experience of a product or solution, the method being performed by an analysis device, wherein the method comprises:
- for a type of participant interacting with the product or solution, defining (610) multiple input features, wherein an input feature is a measurable quantity of an interaction with the product or solution, and wherein the input feature is related to an experience driver that affects an experience of the product or solution for the type of participant;
- collecting (620) multiple training records, wherein a training record comprises values of the multiple input features and an overall experience score;
- based on the multiple training records, training (630) a machine learning model to infer the overall experience score from at least the multiple input features;
- deriving (640) importance weights of the multiple input features from the trained machine learning model, wherein an importance weight of an input feature indicates an importance assigned by the machine learning model to the input feature for inferring the overall experience score;
- outputting (650) importance weights of respective input features to provide feedback about a relative importance of the respective input features on the experience of the type of participant interacting with the product or solution.

2. The method (600) of claim 1, wherein the product or solution is a medical product or solution, for example comprising a medical imaging system, and the type of participant is a patient or a medical professional.

3. The method (600) of any preceding claim, further comprising: obtaining values of the multiple input features and computing an impact score on the experience from the importance weights and the values of the multiple input features.

4. The method (600) of any preceding claim, further comprising determining importance weights for a particular experience driver by normalizing the importance weights derived for a subset of input features related to the particular experience driver, and/or obtaining values for the subset of input features and computing an impact score on the particular experience driver from the values and importance weights for the subset of input features.

5. The method (600) of any preceding claim, wherein the machine learning model is a non-linear model, for example an ensemble of decision trees.

6. The method (600) of any preceding claim, wherein collecting the multiple training records comprises normalizing the multiple input features to a common scale.

7. The method (600) of any preceding claim, further comprising collecting additional training records; tuning the machine learning model; and deriving updated importance weights from the tuned machine learning model.

8. The method (600) of any preceding claim, wherein an input feature corresponds to a number of times a given action is performed by the type of participant or by the product or solution in an interaction with the product or solution.

9. The method (600) of any preceding claim, wherein a training record corresponds to a time period, and wherein a value of an input feature and/or an experience driver and/or an overall experience score of the training record is an aggregate value over the time period.

10. The method (600) of any preceding claim, comprising testing the machine learning model on validation records to obtain an accuracy of the machine learning model, and outputting the accuracy to the user of the analysis system as an indicator of a predictability of the overall experience score from the multiple input features.

11. The method (600) of any preceding claim, wherein the machine learning model is trained to use one or more confounding features in addition to the multiple input features for inferring the overall experience score.

12. The method (600) of any preceding claim, comprising extracting values of at least one of the multiple input features from log data of the product or solution.

13. The method (600) of any preceding claim, wherein an input feature represents hardware data, software data, a survey outcome, or other qualitative data.

14. An analysis device (1100) for use in analyzing an experience of a product or solution, wherein, for a type of participant interacting with the product or solution, multiple input features are defined, wherein an input feature is a measurable quantity of an interaction with the product or solution, and wherein the input feature is related to an experience driver that affects an experience of the product or solution for the type of participant, wherein the device comprises:
- a memory (1130) for storing multiple training records, wherein a training record comprises values of the multiple input features and an overall experience score;
- a processor (1120) configured to:
- collect the multiple training records;
- based on the multiple training records, train a machine learning model to infer the experience score from at least the multiple input features;
- derive importance weights of the multiple input features from the trained machine learning model, wherein an importance weight of an input feature indicates an importance assigned by the machine learning model to the input feature for inferring the overall experience score;
- output importance weights of respective input features to provide feedback about a relative importance of the respective input features on the experience of the type of participant of the product or solution.

15. A computer readable medium (1000) comprising transitory or non-transitory data (1020) representing instructions to cause a processor system to perform the method according to any one of claims 1-13.
